# EUROPÄISCHE PATENTSCHRIFT

(11) **EP 2 923 726 B1**
(45) Veröffentlichungstag und Bekanntmachung des Hinweises auf die Patenterteilung: **11.01.2017**
(21) Anmeldenummer: 15000499.2
(22) Anmeldetag: 20.02.2015
(51) Int. Cl.: A61N 1/04, A61N 1/36, A61B 5/00, H04R 25/00

(54) **VERFAHREN ZUR HERSTELLUNG EINER STIMULATIONSVORRICHTUNG**
METHOD FOR PRODUCING A STIMULATION DEVICE
PROCÉDÉ DE FABRICATION D'UN DISPOSITIF DE STIMULATION

(30) Priorität: 18.03.2014 DE 102014003759
(43) Veröffentlichungstag der Anmeldung: 30.09.2015
(73) Patentinhaber: cerboMed GmbH, 91052 Erlangen (DE)
(72) Erfinder: Zschaeck, Thomas, 90427 Nürnberg (DE); Frenkel, Wolf Gerhard, 72514 Inzigkofen-Engelswies (DE); Hartlep, Andreas, 83607 Holzkirchen (DE)
(74) Vertreter: Gosdin, Michael

(56) Entgegenhaltungen:
- EP-A1- 1 788 009
- EP-A1- 2 227 041
- WO-A1-2011/000375
- US-A1- 2005 222 823

## Beschreibung

Die Erfindung betrifft ein Verfahren zur Herstellung einer Stimulationsvorrichtung zur Aufbringung eines elektrischen, magnetischen, optischen, akustischen oder mechanischen Impulses auf die Oberfläche eines Abschnittes eines Ohres des menschlichen Körpers, wobei die Stimulationsvorrichtung einen Trägerkörper und mindestens ein stimulationserzeugendes Element aufweist.

Eine solche Stimulationsvorrichtung wird beispielsweise benötigt, um einen elektrischen transkutanen Stimulationsreiz auf einen Nerv, insbesondere den Vagusnerv, auszuüben, d. h. ohne die Vorrichtung bzw. Teile derselben in den Körper des Patienten implantieren zu müssen. Hierzu wird die Stimulationsvorrichtung beispielsweise in das Ohr des Patienten eingesetzt.

Eine derartige Stimulationsvorrichtung ist beispielsweise aus der DE 10 2012 014 764 A1 bekannt. Die Vorrichtung hat sich bewährt, um mittels der Methode der transkutanen elektrischen Nervenstimulation Impulsströme verschiedener Stromformen, Amplituden, Impulsdauern und Frequenzen durch die Haut hindurch an verschiedenen Nerven zu applizieren und so verschiedene Krankheiten zu behandeln. Eine ähnliche Lösung ist aus der US 4 267 838 bekannt.

Ferner ist aus WO 2011/000375 ein Ohrstöpsel mit Oberflächenelektroden bekannt.

Es hat dabei Vorteile, wenn die die Hautoberfläche kontaktierenden Bereiche der Stimulationsvorrichtung möglichst gut der Oberflächentopographie, d. h. der Form der Hautoberfläche des zu stimulierenden Bereichs, angepasst sind.

Bei der vorbekannten Lösung wird dies durch eine entsprechende Einstellmöglichkeit der Vorrichtung bewerkstelligt. Möglich, allerdings aufwändig und somit teuer, ist es auch, einen Abguss des individuellen Ohrs anzufertigen und hiernach eine Otoplastik herzustellen.

Der Erfindung liegt die **Aufgabe** zugrunde, ein Verfahren zur Herstellung einer Stimulationsvorrichtung der eingangs genannten Art vorzuschlagen, mit der es möglich ist, die Stimulationsvorrichtung optimal an die Bedürfnisse eines Trägers anpassen zu können, wobei allerdings günstigere Herstellungskosten realisiert werden sollen, als dies bislang der Fall ist. Dabei soll insbesondere eine im Sinne dieser Aufgabenstellung vorteilhafte Stimulationsvorrichtung herstellbar sein, die zur transkutanen Stimulation des Vagusnervs ins Ohr des Trägers eingesetzt bzw. am Ohr platziert wird.

Die **Lösung** dieser Aufgabe durch die Erfindung ist dadurch gekennzeichnet, dass das Verfahren die Schritte aufweist:
a) Scannen der Oberflächentopographie zumindest eines Teil des Ohres des Trägers der Stimulationsvorrichtung und Erzeugung eines Datensatzes für die Beschreibung der Oberflächentopographie;
b) Anordnen des mindestens einen stimulationserzeugenden Elements in einer Produktionsvorrichtung;
c) Herstellung des Trägerkörpers in der Produktionsvorrichtung durch schichtweises, dreidimensionales Aufbringen des Materials des Trägerkörpers mittels eines Druckkopfs gemäß dem erstellten Datensatz (also im 3-D-Druck) oder durch schichtweises, dreidimensionales Aufschmelzen und Verfestigenlassen des Materials des Trägerkörpers mittels eines Laserstrahls gemäß dem erstellten Datensatz, wobei das mindestens eine stimulationserzeugende Element zumindest teilweise vom Material des Trägerkörpers ummantelt wird.

Bevorzugt ist dabei an eine Stimulationsvorrichtung gedacht, die zur Stimulation des Vagusnervs in das Ohr des Trägers eingesetzt oder an diesem angeordnet wird. Hierbei sind die stimulationserzeugenden Elemente zumeist Elektroden, über die eine elektrische Stimulation erfolgen kann.

Das Anordnen des mindestens einen stimulationserzeugenden Elements in der Produktionsvorrichtung gemäß obigem Schritt b) kann dabei so erfolgen, dass das mindestens eine stimulationserzeugende Element durch schichtweises, dreidimensionales Aufbringen des Materials des stimulationserzeugenden Elements mittels des Druckkopfs (oder mittels eines weiteren Druckkopfs) hergestellt wird. Hiernach würde also auch das stimulationserzeugende Element selber im genannten 3-D-Druck hergestellt werden. Da es möglich ist, auch elektrisch leitfähige Materialeien im 3-D-Druck zu verarbeiten, insbesondere auch Metalle, eröffnet dies die Möglichkeit, das Stimulations-Element mit einer individuell optimierten Gestalt zu erzeugen.

Vor der Herstellung des mindestens einen stimulationserzeugenden Elements kann mindestens ein elektrisches Kabel in der Produktionsvorrichtung platziert werden, wobei beim Herstellen des mindestens einen stimulationserzeugenden Elements das Material desselben mit einem leitfähigen Abschnitt des mindestens einen elektrischen Kabels elektrisch leitend verbunden wird.

Das mindestens eine stimulationserzeugende Element kann auch aus einem nichtleitenden, porösen Material gefertigt werden, insbesondere aus Polyurethan, wobei dann in das Material des stimulationserzeugenden Elements vor der Benutzung der Vorrichtung eine elektrisch leitfähige Flüssigkeit oder ein elektrisch leitfähiges Gel eingebracht wird, um das stimulationserzeugende Element elektrisch leitend zu machen.

Möglich ist es alternativ aber auch, dass gemäß obigem Schritt b) mindestens ein vorgefertigtes stimulationserzeugendes Element, beispielsweise eine Elektrode, in der Produktionsvorrichtung angeordnet wird, die dann gemäß obigem Schritt c) entsprechend vom Material des Trägerkörpers abschnittsweise umgeben wird.

Das schichtweise, dreidimensionale Aufbringen des Materials des Trägerkörpers kann dabei so erfolgen, dass das mindestens eine stimulationserzeugende Element um einen definierten Überstand aus der Oberfläche des Trägerkörpers hervorsteht. Hierdurch kann die Kontaktqualität des stimulationserzeugenden Elements mit der Hautoberfläche optimiert werden.

Nach obigem Schritt b) und vor obigem Schritt c) kann ferner eine Halterung in der Produktionsvorrichtung angeordnet werden, um das mindestens eine stimulationserzeugende Element während des obigen Schritts c) in Position zu halten. Diese Halterung ist also ein Hilfselement, um den Produktionsprozess zu stabilisieren. Die Halterung kann nach der Herstellung des Trägerkörpers in der Stimulationsvorrichtung verbleiben (und dabei beispielsweise vollständig vom Material des Trägerkörpers ummantelt sein); es kann aber auch wieder aus dem Trägerkörper entfernt (herausgezogen) werden.

Weiterhin kann vorgesehen werden, dass an den Trägerkörper ein Halteelement angebracht wird, das zum Fixieren, insbesondere zum Festklemmen, des Trägerkörpers am oder im Körper des Trägers ausgebildet ist. Die Herstellung des Halteelements kann dabei wieder in der Produktionsvorrichtung erfolgen, indem das Halteelement - insbesondere in einstückiger Bauweise zusammen mit dem Trägerelement - durch schichtweises, dreidimensionales Aufbringen des Materials des Halteelements mittels des Druckkopfs (oder eines anderen Druckkopfs) im 3-D-Druck hergestellt wird. Allerdings ist es auch hier alternativ möglich, dass das Halteelement als separat vorgefertigtes Bauteil mit dem Trägerkörper verbunden wird.

Wenngleich sich das vorgeschlagene Verfahren für Stimulationsvorrichtungen beliebiger Art eignet, die an dem oder im Körper des Patienten an- oder eingesetzt werden, um in beliebiger Weise einen Reiz auf den Körper auszuüben, eignet sich das vorgeschlagene Verfahren besonders für die Herstellung einer Stimulationsvorrichtung zur Aufbringung eines transkutanen elektrischen Stimulationsreizes auf die Oberfläche eines Abschnittes des menschlichen Ohrs, wobei das stimulationserzeugende Element in diesem Falle insbesondere eine Elektrode ist.

Für die Fertigung des Trägerkörpers kann ein biokompatibles Material verwendet werden, das sich im 3-D-Druck verarbeiten lässt. Besonders bevorzugt ist hier an einen Kunststoff auf Methacrylatharzbasis gedacht, was sich gut für medizinische Produkte eignet.

Wie erläutert, können konventionelle Metall-Elektroden als stimulationserzeugende Elemente eingesetzt werden.

Im Rahmen des Scannens der Oberflächentopographie kann präzise die individuelle Form beispielsweise der Cymba Conchae des Ohrs erfasst werden, so dass der anschließend hergestellte Trägerkörper kongruent zur Form der Cymba Conchae ist.

Am Trägerkörper kann, wie erwähnt, nach den Gegebenheiten ein Halteelement angeordnet werden. Im Falle einer erfindungsgemäß individuell hergestellten Otoplastik kann zwar auch ohne eine weitergehende Verankerung (also ohne Halteelement) gearbeitet werden, es können aber auch diverse Ausgestaltungen eines Halteelements vorgesehen werden. Das Halteelement kann beispielsweise von vorne bzw. von unten mit einem Antihelix-Ausleger versehen sein. Es ist auch eine Verankerung unter der Antihelix nach vorne bzw. nach unten möglich, die dann im Bogen zurück über die Helixwurzel und im Ringschluss wieder zurück zur Cymba Conchae führt. Weiter möglich ist auch eine Verankerung unter der Antihelix nach vorne bzw. unten, die dann im Bogen zurück unter dem Tragus hindurch und im Ringschluss wieder zurück zur Cymba Conchae führt. Schließlich ist eine Verankerung nach vorne und hinter das Ohr im Sinne eines HdO-Bügels (Hinter-dem-Ohr-Bügel) möglich.

Das Halteelement kann auch so gefertigt werden, dass es den Trägerkörper mit seiner mindestens einen Elektrode bei bestimmungsgemäßer Benutzung mit definierter Vorspannung gegen die jeweilige anatomische Struktur drückt, um insgesamt einen guten Halt der Vorrichtung und eine ausreichende Kontaktqualität sicherzustellen.

Im Falle vorgefertigter Elektroden können beispielsweise Titan-Elektroden verwendet werden. Diese können während des Fertigungsprozesses des Trägerkörpers (s. obigen Schritt c)) mittels einer Haltevorrichtung formstabil in der korrekten Ausrichtung gehalten werden. Bei der Haltevorrichtung kann es sich um eine elektrisch isolierende Quertraverse handeln, die entweder im Trägerkörper verbleibt oder nach dem Produzieren derselben seitlich herausgezogen wird.

Im Falle von Elektroden, die erst im Herstellungsprozess per 3-D-Druck gefertigt werden, kann vorgesehen werden, dass zunächst eine erste Schicht der Elektrode gedruckt wird und dann das abisolierte Kabelende mit Zugentlastung auf das Elektrodenmaterial aufgelegt wird; dann erfolgt der weitere Druck der Elektrode, wodurch das Kabelende von leitfähigem Material umhüllt und gut kontaktiert ist.

Der Vorteil bei der Fertigung der Elektrode selber per 3-D-Druck ist, dass die Elektrodenoberfläche frei an die individuelle Ohr-Krümmung bzw. -Wölbung angepasst werden kann. Dadurch werden gleichzeitig Tragekomfort und Kontaktqualität der Stimulation verbessert.

Hinsichtlich eines elektrischen Anschlusses können fest angeschlossene Litzen vorgesehen werden, die bereits direkt in das Kabel zur Stimulationsvorrichtung übergehen oder mit einer (zweipoligen) Steckbuchse als Schnittstelle versehen sind, die unter Freilassung einer Einstecköffnung umspritzt bzw. umdruckt wird.

Die Elektroden können als flächiges, leicht balliges Oval mit abgerundeten Rändern im Sinne eines Falzes ausgebildet sein. Der Falz stellt eine formschlüssige Verbindung mit dem Material des Trägerkörpers sicher.

Die Elektroden können relativ groß ausgebildet sein (im Vergleich mit vorbekannten Lösungen, wie oben erwähnt, auch deutlich größer), so dass dadurch die Stromdichte, die in die Haut übertragen werden muss, sinkt. Dies wiederum optimiert ebenfalls den Tragekomfort.

Möglich ist es auch, dass bei der Fertigung der Stimulationsvorrichtung eine Auswahl an verschiedenen Elektroden bereit steht, wobei die Elektroden dann für den konkreten Fall ausgewählt werden.

Beim Herstellen des Trägerkörpers, also insbesondere per 3-D-Druck, können somit die Form und die Wölbung der Cymba Conchae patientenspezifisch berücksichtigt werden.

Wie bereits erwähnt, kann für die Elektroden ein Überstand aus der Oberfläche des Trägerkörpers vorgesehen werden, beispielsweise um einige Zehntel Millimeter. Dadurch schmiegen sie sich besser an die Hautoberfläche an. Außerdem kann dann auch zwischen Otoplastik und Haut Luft zirkulieren, was einem Feuchtigkeits- und Wärmestau vorbeugt.

Das Kabel wird vom Trägerkörper entsprechend abgeführt. Die Kabelführung kann direkt nach unten über die Incisura intertragica erfolgen oder hinter dem Ohr nach unten, was unter dem Gesichtspunkt der Zugentlastung vorteilhaft ist.

Werden, wie alternativ genannt, die Elektroden ebenfalls im 3-D-Druck hergestellt, kann hierfür ein elektrisch leitfähiger, biokompatibler Kunststoff vorgesehen werden.

Dabei können zunächst die blanken Enden der Litze (des Kabels) in die Produktionsvorrichtung eingelegt und fixiert werden, so dass diese beim 3-D-Druck innerhalb der jeweiligen Elektrode zu liegen kommen. Dann erfolgt das 3-D-Drucken der elektrisch leitfähigen Elektroden mit leitendem Kontakt zu den Litzenenden, bevorzugt aus biokompatiblem, leitfähigem und druckfähigem Material. Die leitfähigen Elektroden haben zueinander keinen leitenden Kontakt, wohl aber später zur anliegenden Haut.

Die Haut-Kontaktfläche der Elektroden kann zur Verringerung der Stromdichte einige Quadratmillimeter betragen.

Dann erfolgt das 3-D-Drucken der restlichen Otoplastik (d. h. des Trägerkörpers) bis zur vollständigen Kontur aus nicht leitfähigem, ebenfalls allerdings biokompatiblem Material.

Dabei kann der per Scan erzeugte Datensatz so manipuliert werden, dass auch die leitfähigen gedruckten Elektroden einige Zehntel Millimeter gegenüber der restlichen Otoplastik (d. h. gegenüber dem Trägerkörper) vorstehen, was einerseits den Stromkontakt zu Haut verbessert, andererseits aber auch eventuellen Kurzschlüssen durch ein Kontaktgel bzw. eine leitfähige Flüssigkeit vorbeugt und schließlich den Tragekomfort durch bessere Luftzirkulation unter der Elektrode merklich verbessert.

Das genannte Halteelement kann sowohl aus isolierendem als auch aus leitfähigem Material bestehen. Es kann im gegebenen Falle vorteilhaft sein, wenn der Strom außer in der Cymba Conchae auch an anderen Stellen im übrigen Ohr appliziert wird. Bei Ringschluss (Ringform) der Verankerung müsste allerdings eine isolierende Zone zwischengedruckt bzw. zwischengespritzt werden.

Die elektrische Leitfähigkeit der Elektroden kann auch durch ein offenporigporöses, nicht leitendes Material hergestellt werden, wenn nämlich dieses Material mit einer leitfähigen Flüssigkeit getränkt wird, die sich in den Poren gut hält.

Vorteilhaft ist es, wenn die Porosität mit gleichzeitiger Weichheit kombiniert wird, mit einem randseits umlaufenden Wulst, beispielsweise aus Polyurethan-Schaum, der die Flüssigkeit nach außen hin abdichtet.

Wenn das poröse Material indes hart ist, kann beispielsweise ein separater Ring, z. B. aus Polyurethan-Schaum, zur Bildung des genannten umlaufenden Wulstes eingesetzt werden.

Primär vorgesehen ist erfindungsgemäß also, dass jedenfalls der Trägerkörper im 3-D-Druck hergestellt wird, und zwar aus biokompatiblem Material, das sich gut im 3-D-Druck verarbeiten lässt. Da sich auch andere Materialien, insbesondere Metalle, im 3-D-Druck verarbeiten lassen, bietet sich auch das Drucken der Elektroden an, die dann vorteilhaft in der optimalen Form gefertigt werden können.

Nach einer Alternative wird ein Kunststoff-Granulat bei der Fertigung des Trägerkörpers eingesetzt, das dreidimensional schichtweise mittels eines Laserstrahls aufgeschmolzen wird und das sich dann wieder verfestigt. Auch somit kann die individuelle Form des Trägerkörpers anhand des gescannten Datensatzes gewonnen werden.

In der Zeichnung ist ein Ausführungsbeispiel der Erfindung dargestellt. Es zeigen:
- Fig. 1: in perspektivischer Darstellung eine Stimulationsvorrichtung zur transkutanen Stimulation, die in ein Ohr eines Menschen einsetzbar ist,
- Fig. 2: die Draufsicht auf eine ähnliche Stimulationsvorrichtung,
- Fig. 3: den Schnitt A-B gemäß Fig. 2 und
- Fig. 4: schematisch in perspektivischer Darstellung eine Produktionsvorrichtung, mit der eine Stimulationsvorrichtung gefertigt werden kann.

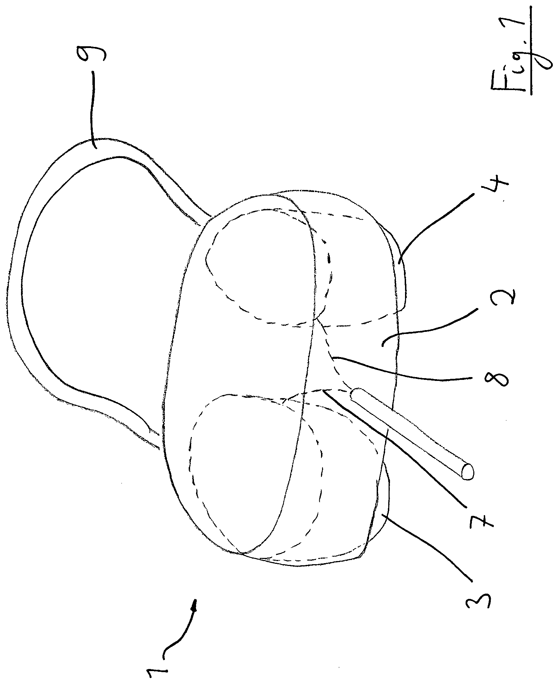

In Fig. 1 ist eine Stimulationsvorrichtung 1 zu sehen, die zur transkutanen Stimulation des Vagusnervs in der Pinna eines Patienten platziert wird. Zur generellen Technologie wird auf die DE 10 2012 014 764 A1 der Patentanmelderin ausdrücklich Bezug genommen. Die Stimulationsvorrichtung 1 weist einen Trägerkörper 2 auf, in den zwei Elektroden 3 und 4 integriert sind. Über zwei Kabel 7 und 8 stehen die Elektroden 3, 4 mit einer nicht dargestellten Steuervorrichtung in Verbindung.

Damit die Stimulationsvorrichtung 1 im Ohr, mit den Elektroden 3, 4 das Cavum conchae kontaktierend, Halt findet, ist ein Haltelement 9 vorgesehen, das an dem Trägerkörper 2 angebracht ist. Das Halteelement 9 ist in Fig. 1 nur schematisch dargestellt. Es kann ringförmig geschlossen sein oder auch ein oder zwei Arme aufweisen, die elastisch an der Kontur des Ohrs Halt finden und so die Stimulationsvorrichtung 1 im Ohr fixieren.

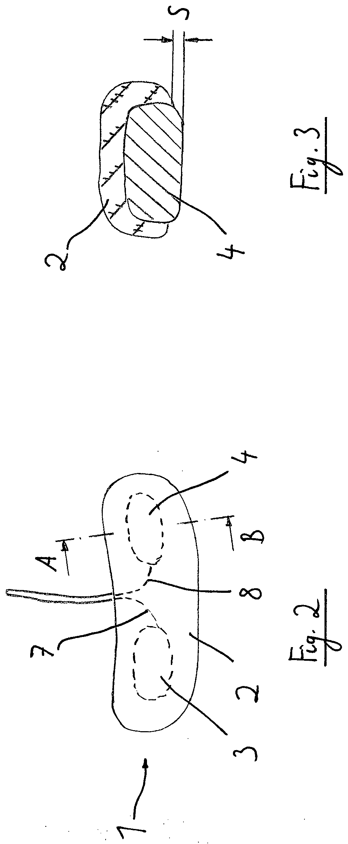

In den Figuren 2 und 3 kann gesehen werden, dass die Elektroden 3, 4 aus der Basisgeometrie des Trägerkörpers 2 etwas überstehen, und zwar um einen Überstand s (s. Fig. 3). Damit wird ein zuverlässiger Kontakt der Elektroden 3, 4 bei ins Ohr eingesetzter Stimulationsvorrichtung sichergestellt.

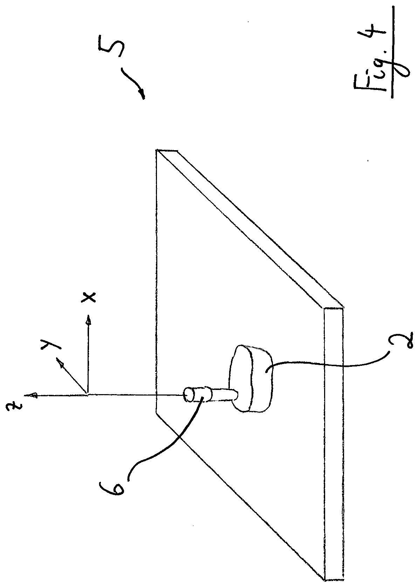

Wesentlich ist die Herstellungsweise der Stimulationsvorrichtung 1, die in Fig. 4 schematisch dargestellt ist. Zum Einsatz kommt das an sich bekannte dreidimensionale Druckverfahren (3-D-Druck), bei dem schichtweise eine dreidimensionale Struktur aus einem Material aufgebaut wird, das über einen Druckkopf 6 einer Produktionsvorrichtung 5 ausgebracht wird. Dabei wird bei vorgegebener z-Koordinate (s. Fig. 4), die durch die Koordinaten x und y definierte Ebene linienweise abgefahren und über den Druckkopf 6 an der Stelle eine definierte kleine Materialmenge ausgebracht, wo sich beim herzustellenden Trägerkörper 2 Material befinden muss.

Ist diese Ebene abgefahren, wird der Druckkopf 6 in z-Richtung um einen definierten Betrag weiter verschoben und die Aufbringung in der neuen x-y-Ebene beginnt.

Dies wird so lange wiederholt, bis der Trägerkörper 2 hergestellt ist.

Demgemäß beginnt das Herstellungsverfahren für die Stimulationsvorrichtung durch ein Scannen der Oberflächentopographie beispielsweise des Ohrs, insbesondere im Bereich der Cymba conchae. Hieraus wird ein Datensatz erzeugt, der die Oberflächentopographie beschreibt.

Dann werden die Elektroden 3, 4 in der Produktionsvorrichtung 5 angeordnet.

Schließlich erfolgt die Herstellung des Trägerkörpers 2 in der Produktionsvorrichtung 5 durch das besagte schichtweise, dreidimensionale Aufbringen des Materials des Trägerkörpers 2 mittels des Druckkopfs 6. Hierbei liegt der aus dem Scan stammende Datensatz zugrunde. Die Elektroden 3, 4 werden hierbei teilweise ummantelt, wie in Fig. 3 am besten zu sehen. Der Datensatz kann gegebenenfalls manipuliert werden, um die Form des Trägerkörpers teilweise im Sinne einer vorteilhaften medizinischen Applikation zu verändern.

Über den Druckkopf 6, oder alternativ auch über einen weiteren, nicht dargestellten Druckkopf, können zunächst die Elektroden 3, 4 per 3-D-Drcuk erzeugt werden, die dann im weiteren Verfahrensverlauf vom Material des Trägerkörpers 2 teilweise ummantelt werden, bis die dargestellte Struktur erzeugt ist.

### Bezugszeichenliste:

- 1: Stimulationsvorrichtung
- 2: Trägerkörper
- 3: stimulationserzeugendes Element (Elektrode)
- 4: stimulationserzeugendes Element (Elektrode)
- 5: Produktionsvorrichtung
- 6: Druckkopf
- 7: Kabel
- 8: Kabel
- 9: Halteelement

- s: Überstand
- x: Koordinate
- y: Koordinate
- z: Koordinate

## Patentansprüche

1. Verfahren zur Herstellung einer Stimulationsvorrichtung (1) zur Aufbringung eines elektrischen, magnetischen, optischen, akustischen oder mechanischen Impulses auf die Oberfläche eines Abschnittes eines Ohres des menschlichen Körpers, wobei die Stimulationsvorrichtung (1) einen Trägerkörper (2) und mindestens ein stimulationserzeugendes Element (3, 4) aufweist, wobei das Verfahren die folgenden Schritte aufweist:
a) Scannen der Oberflächentopographie zumindest eines Teil des Ohres des Trägers der Stimulationsvorrichtung (1) und Erzeugung eines Datensatzes für die Beschreibung der Oberflächentopographie;
b) Anordnen des mindestens einen stimulationserzeugenden Elements (3, 4) in einer Produktionsvorrichtung (5);
c) Herstellung des Trägerkörpers (2) in der Produktionsvorrichtung (5) durch schichtweises, dreidimensionales Aufbringen des Materials des Trägerkörpers (2) mittels eines Druckkopfs (6) gemäß dem erstellten Datensatz oder durch schichtweises, dreidimensionales Aufschmelzen und Verfestigenlassen des Materials des Trägerkörpers (2) mittels eines Laserstrahls gemäß dem erstellten Datensatz,
**dadurch gekennzeichnet, dass**
das mindestens eine stimulationserzeugende Element (3, 4) derart in Schritt b) in der Produktionsvorrichtung angeordnet wird, dass es in Schritt c) zumindest teilweise vom Material des Trägerkörpers (2) ummantelt wird.

2. Verfahren nach Anspruch 1, **dadurch gekennzeichnet, dass** das Anordnen des mindestens einen stimulationserzeugenden Elements (3, 4) in der Produktionsvorrichtung (5) gemäß Schritt b) von Anspruch 1 erfolgt, indem das mindestens eine stimulationserzeugende Element (3, 4) durch schichtweises, dreidimensionales Aufbringen des Materials des stimulationserzeugenden Elements (3, 4) mittels des Druckkopfs (6) hergestellt wird.

3. Verfahren nach Anspruch 2, **dadurch gekennzeichnet, dass** vor der Herstellung des mindestens einen stimulationserzeugenden Elements (3, 4) mindestens ein elektrisches Kabel (7, 8) in der Produktionsvorrichtung (5) platziert wird, wobei beim Herstellen des mindestens einen stimulationserzeugenden Elements (3, 4) das Material desselben mit einem leitfähigen Abschnitt des mindestens einen elektrischen Kabels (7, 8) elektrisch leitend verbunden wird.

4. Verfahren nach Anspruch 2 oder 3, **dadurch gekennzeichnet, dass** das mindestens eine stimulationserzeugende Element (3, 4) aus einem nichtleitenden, porösen Material gefertigt wird, insbesondere aus Polyurethan, wobei in das Material des stimulationserzeugenden Elements (3, 4) eine elektrisch leitfähige Flüssigkeit oder ein elektrisch leitfähiges Gel eingebracht wird, um das stimulationserzeugende Element (3, 4) elektrisch leitend zu machen.

5. Verfahren nach Anspruch 1, **dadurch gekennzeichnet, dass** gemäß Schritt b) von Anspruch 1 mindestens ein vorgefertigtes stimulationserzeugendes Element (3, 4) in der Produktionsvorrichtung (5) angeordnet wird.

6. Verfahren nach einem der Ansprüche 1 bis 5, **dadurch gekennzeichnet, dass** das schichtweise, dreidimensionale Aufbringen des Materials des Trägerkörpers (2) so erfolgt, dass das mindestens eine stimulationserzeugende Element (3, 4) um einen definierten Überstand (s) aus der Oberfläche des Trägerkörpers (2) hervorsteht.

7. Verfahren nach einem der Ansprüche 1 bis 6, **dadurch gekennzeichnet, dass** nach Schritt b) und vor Schritt c) gemäß Anspruch 1 eine Halterung in der Produktionsvorrichtung (5) angeordnet wird, um das mindestens eine stimulationserzeugende Element (3, 4) während des Schritts c) gemäß Anspruch 1 in Position zu halten.

8. Verfahren nach einem der Ansprüche 1 bis 7, **dadurch gekennzeichnet, dass** an den Trägerkörper (2) ein Halteelement (9) angebracht wird, das zum Fixieren, insbesondere zum Festklemmen, des Trägerkörpers (2) am oder im Körper des Trägers ausgebildet ist.

9. Verfahren nach Anspruch 8, **dadurch gekennzeichnet, dass** die Herstellung des Halteelements (9) in einstückiger Bauweise zusammen mit dem Trägerkörper (2) in der Produktionsvorrichtung (5) erfolgt, indem das Halteelement (9) durch schichtweises, dreidimensionales Aufbringen des Materials des Halteelements (9) mittels des Druckkopfs (6) hergestellt wird.

10. Verfahren nach Anspruch 8, **dadurch gekennzeichnet, dass** das Halteelement (9) als separat vorgefertigtes Bauteil mit dem Trägerkörper (2) verbunden wird.

## Claims

1. Method for producing a stimulation device (1) for the application of an electrical, magnetic, optical, acoustical or mechanical impulse onto the surface of a section of an ear of a human body, wherein the stimulation device (1) comprises a carrier body (2) and at least one stimulating element (3, 4), wherein the method comprises the following steps:
a) Scanning of the surface topography of at least a part of the ear of the user of the stimulation device (1) and creation of a data set for the description of the surface topographie;
b) Arrangement of the at least one stimulating element (3, 4) in a production device (5);
c) Production of the carrier body (2) in the production device (5) by three-dimensional application of the material of the carrier body (2) in layers by means of printing head (6) according to the generated data set or by three-dimensional melting and solidification of the material of the carrier body (2) in layers by means of a laser beam according to the generated data set,
**characterized in that**
the at least one stimulating element (3, 4) is arranged in the production device in such a manner in step b) that it is at least partially encased by the material of the carrier body (2) in step c).

2. Method according to claim 1, **characterized in that** the arrangement of the at least one stimulating element (3, 4) in the production device (5) according to step b) of claim 1 takes place by three-dimensional application of the material of the stimulating element (3, 4) in layers by means of the printing head (6).

3. Method according to claim 2, **characterized in that** prior to the production of the at least one stimulating element (3, 4) at least one electrical cable (7, 8) is arranged in the production device (5), wherein at the production of the at least one stimulating element (3, 4) the material of the same is connected with a conductive section of the at least one electrical cable (7, 8) in an electrical conductive manner.

4. Method according to claim 2 or 3, **characterized in that** the at least one stimulating element (3, 4) is produced from a non-conductive porous material, especially from polyurethane, wherein an electrical conductive fluid or an electrical conductive gel is applied into the material of the stimulating element (3, 4) to make the stimulating element (3, 4) electrically conductive.

5. Method according to claim 1, **characterized in that** according to step b) of claim 1 at least one pre-fabricated stimulating element (3, 4) is arranged in the production device (5).

6. Method according to one of claims 1 to 5, **characterized in that** the three-dimensional application of the material of the carrier body (2) in layers takes place in such a manner that the at least one stimulating element (3, 4) protrudes by a defined protrusion (s) from the surface of the carrier body (2).

7. Method according to one of claims 1 to 6, **characterized in that** after step b) and prior to step c) of claim 1 a holding is arranged in the production device (5) to hold the at least one stimulating element (3, 4) during step c) of claim 1 in position.

8. Method according to one of claims 1 to 7, **characterized in that** a holding element (9) is affixed to the carrier body (2) which is designed for the fixation, especially for clamping, of the carrier body (2) at or in the body of the wearer.

9. Method according to claim 8, **characterized in that** the production of the holding element (9) takes place in a one-piece design together with the carrier body (2) in the production device (5) by production of the holding element (8) in a three-dimensional application of the material of the holding element (9) in layers by means of the printing head (6).

10. Method according to claim 8, **characterized in that** the holding element (9) being a separately pre-produced part is connected with the carrier body (2).

## Revendications

1. Procédé destiné à fabriquer un dispositif de stimulation (1) pour l'application d'une impulsion électrique, magnétique, optique, acoustique ou mécanique sur la surface d'une partie d'une oreille du corps humain, le dispositif de stimulation (1) comportant un corps de support (2) et au moins un élément (3, 4) générant des stimulations,
le procédé comportant les étapes suivantes :
a) scanner la topographie superficielle d'au moins une partie de l'oreille du porteur du dispositif de stimulation (1) et générer un ensemble de données pour la description de la topographie superficielle ;
b) disposer l'au moins un élément (3, 4) générant des stimulations dans un dispositif de production (5) ;
c) fabriquer le corps de support (2) dans le dispositif de production (5) par application tridimensionnelle couche par couche de la matière du corps de support (2) à l'aide d'une tête d'impression (6) conformément à l'ensemble de données établi ou par fusion tridimensionnelle, couche par couche et en laissant durcir la matière du corps de support (2) à l'aide d'un rayon laser selon l'ensemble de données établi,
**caractérisé en ce que**
dans l'étape b), on dispose l'au moins un élément (3, 4) générant des stimulations dans le dispositif de production de telle sorte que dans l'étape c), il soit enveloppé au moins partiellement par la matière du corps de support (2).

2. Procédé selon la revendication 1, **caractérisé en ce que** la disposition de l'au moins un élément (3, 4) générant des stimulations dans le dispositif de production (5) s'effectue selon l'étape b) de la revendication 1 **en ce qu'**on fabrique l'au moins un élément (3, 4) générant des stimulations par application tridimensionnelle, couche par couche de la matière de l'élément (3, 4) générant des stimulations à l'aide de la tête d'impression (6).

3. Procédé selon la revendication 2, **caractérisé en ce qu'**avant la fabrication de l'au moins un élément (3, 4) générant des stimulations, on place au moins un câble (7, 8) dans le dispositif de production (5), sachant que lors de la fabrication de l'au moins un élément générant des stimulations (3, 4) on relie de manière conductrice d'électricité la matière de celui-ci avec une partie conductrice de l'au moins un câble (7, 8) électrique.

4. Procédé selon la revendication 2 ou la revendication 3, **caractérisé en ce qu'**on fabrique l'au moins un élément (3, 4) générant des stimulations dans une matière poreuse, non conductrice, notamment en polyuréthane, sachant qu'on introduit dans la matière de l'élément (3, 4) générant des stimulations un liquide conducteur d'électricité ou un gel conducteur d'électricité, pour donner à l'élément (3, 4) générant des stimulations un caractère conducteur d'électricité.

5. Procédé selon la revendication 1, **caractérisé en ce que** selon l'étape b) de la revendication 1, on dispose au moins un élément (3, 4) générant des stimulations préfabriqué dans le dispositif de production (5).

6. Procédé selon l'une quelconque des revendications 1 à 5, **caractérisé en ce que** l'application tridimensionnelle, couche par couche de la matière du corps de support (2) s'effectue de telle sorte que l'au moins un élément (3, 4) générant des stimulations déborde d'une saillie (s) définie de la surface du corps de support (2).

7. Procédé selon l'une quelconque des revendications 1 à 6, **caractérisé en ce qu'**après l'étape b) et avant l'étape c) selon la revendication 1, on dispose une fixation dans le dispositif de production (5) pour maintenir en position un élément (3, 4) générant des stimulations pendant l'étape c) selon la revendication 1.

8. Procédé selon l'une quelconque des revendications 1 à 7, **caractérisé en ce que** sur le corps de support (2) on monte un élément de retenue (9) qui est conçu pour fixer, notamment pour coincer le corps de support (2) sur ou dans le corps du porteur.

9. Procédé selon la revendication 8, **caractérisé en ce que** la fabrication de l'élément de retenue (9) s'effectue en structure monobloc, ensemble avec le corps de support (2) dans le dispositif de production (5) **en ce qu'**on fabrique l'élément de retenue (9) par application tridimensionnelle, couche par couche de la matière de l'élément de retenue (9) à l'aide de la tête d'impression (6).

10. Procédé selon la revendication 8, **caractérisé en ce qu'**on assemble l'élément de retenue (9) en tant que composant préfabriqué séparément avec le corps de support (2).
